Europäisches Patentamt

European Patent Office

Office européen des brevets

(11) Publication number: **0 045 114**
**B1**

(12) EUROPEAN PATENT SPECIFICATION

(45) Date of publication of patent specification: **13.03.85**

(21) Application number: **81200848.0**

(22) Date of filing: **24.07.81**

(51) Int. Cl.⁴: **B 65 D 88/20,** F 17 B 1/26, A 01 C 3/02

(54) **Container for the storage of gas under low pressure, especially bio gas.**

(30) Priority: **25.07.80 NL 8004293**

(43) Date of publication of application:
**03.02.82 Bulletin 82/05**

(45) Publication of the grant of the patent:
**13.03.85 Bulletin 85/11**

(84) Designated Contracting States:
**AT BE DE FR GB LU NL SE**

(56) References cited:
**GB-A-1 507 021**
**GB-A-2 016 571**
**US-A-2 578 090**
**US-A-2 647 657**
**US-A-3 355 271**

(73) Proprietor: **OOSTWOUDER B.V.**
**Rijksweg 126**
**NL-1756 EJ 't Zand (N.H.) (NL)**

(72) Inventor: **Koedooder, Joannes Clemens Maria**
**Dorpsstraat 54**
**NL-1734 JJ Oude Niedorp (NL)**

(74) Representative: **van Assen, Jan Willem Bernard,**
**Ir.**
**Konijnenlaan 22**
**NL-2243 ER Wassenaar (NL)**

Courier Press, Leamington Spa, England.

## Description

The invention relates to a tank for the storage of gas under low pressure, in particular biogas, which tank is provided with a vessel with at least a gas tight bottom and side wall of solid material, whereby on the inner side of the side wall, substantially near the upper end of the side wall, a movable gas tight inner wall is gas tight fixed, which inner wall comprises a thin, flexible membrane at least in the border part adjacent to the side wall, whereas in the thus formed gas tight gas space of variable volume into which at least one conduit debouches for the discharge of gas.

A gas tank of this type is known from the British patent specification 1.522.780 for the storage of biogas, which is kept under substantially constant pressure in the gas tank. Such biogas is produced on for instance farms or other agricultural installations, where large amounts of waste masses are released, such as stable manure and liquid manure, which contain fermentable organic matter, from which biological gas can be produced by anaerobic fermentation.

Hereby a membrane of flexible butyl rubber is applied, which is inflated into the outside atmosphere by the biological gas. Below the membrane a conical frame work support structure is fastened, which protects the membrane against sagging through at low gas pressure. Such gas cushion-gas tanks have the objection, that the butyl rubber is wholly or partly exposed to sun light, by which this ages quickly and starts to leak, whereas these gas cushions are less suitable for greater dimensions, as they then become very sensitive for wind and have to be anchored in a reliable way.

The object of the invention is providing a gas tank lacking the above indicated objections, which object is reached, in that above the inner wall a gas tight pressure space is formed, which is provided with means for the supply and/or removal of fluid, which means are connected to a pressure source, whereby the inner wall is maintained in a floating condition by the pressure difference between the above lying pressure space and the underlying gas space caused by the pressure source.

In this gas tight pressure space according to the invention by the setting of the pressure source a fluid pressure of such a value is obtained, that the biogas can be stored in the gas space below the downwardly bent inner wall under substantially constant pressure.

The invention will now be further elucidated referring to the accompanying drawing of some embodiments.

Fig. 1 shows an axial cross section over a gas tank according to a first main embodiment of the invention in the starting condition.

Fig. 2 shows a similar view as Fig. 1 during the operation.

Fig. 3 shows a similar view as Fig. 1 of a second main embodiment of the invention.

Fig. 4 shows a similar view as Fig. 2 of the second main embodiment according to Fig. 3.

The drawing shows a gas tank which is provided with a round, cylindrical, vertical silo having a solid side wall 1 and a solid bottom 2, which are both preferably gas tight. On the side wall 1 a gas tight, preferably, conical roof 3 is mounted, which is supported by a number of roof joists 4. Such a silo is known per se and is at this time already applied on a large scale for the storage of fodder for animals, such as mown grass, hay, chopped maize and fodder beets, and so forth.

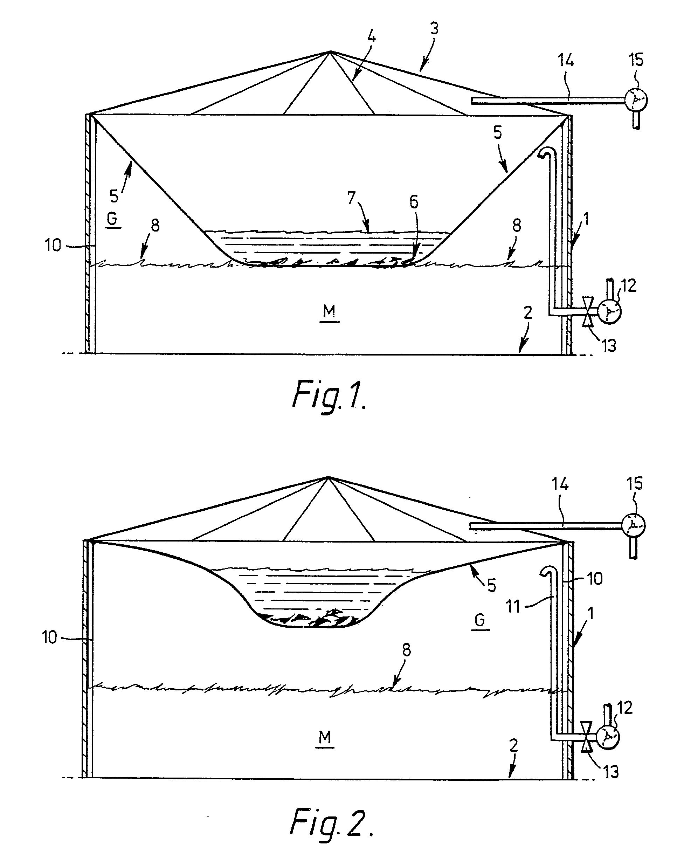

At the inside of the side wall 1 of the silo around the circumference thereof, for instance on the separation between the side wall 1 and the roof 3, a movable, gas tight inner wall 5 is fixedly mounted in a gas tight way. According to the first main embodiment of the invention shown in Figs. 1 and 2, the inner wall 5 exists completely of a membrane 5 of soft and flexible, thin PVC having the form of a body of revolution having a downwardly directed hollow that is symmetrically placed in the silo. A skirt 10 also of soft and flexible PVC is fastened below the membrane 5 and is hanging from the fastening of this membrane 5 to the side wall 1 along this side wall downwardly until near the bottom 2. Thereby the membrane 5 with the skirt 10 forms a gas tight lining for the gas space below the membrane. The membrane 5 is far downwardly bent in the starting position and contains for instance in the hollow an amount of ballast which is composed of for instance stones or pieces of iron 6 and water 7.

Through the side wall 1 or the bottom 2 extends a conduit 11 which stretches until below the membrane 5. This conduit 11 is connected outside the silo 1, 2 to a blower 12 which is adapted for sucking gas out of the gas space. A valve 13 is placed in the conduit 11 before the blower 12. Through the roof 3 extends a conduit 14 which reaches inside the silo 1, 2 until above the membrane 5 and is connected outside the silo 1, 2 to a blower 15 with which the pressure of the fluid F, such as for instance air, in the pressure space above the membrane 5 can be increased to chosen values. In the silo 1, 2 according to the embodiment also an amount of manure M is placed, of which the upper surface is 8. Between the upper surface 8 of the manure and the lower side of the membrane 5 biological gas G is produced by the anaerobic fermentation of the organic manure substances M. This manure M can also be placed in a separate generator (not shown) for biological gas G. As the pressure of this gas G in the gas space increases, the membrane 5 is gradually pressed upward, by which the membrane is displaced from the position according to Figure 1, in which the lower side of the membrane is in contact with the upper side 8 of the manure M, to for instance a new balanced position according to Figure 2, in which the membrane with or without contents floats above the manure surface 8 under influence of the pressure difference and/or the weight loading. In this situation the membrane has preferably taken the shape of approximately a paraboloid, but it can also have other forms, such as that of a half sphere as long as on the one hand there remains enough hollow for containing the ballast which exerts a downward pressure on the membrane, as the proper weight of the membrane is relatively small, and this is not able to guarantee without pressure difference a sufficient pressure of the gas G in the gas space.

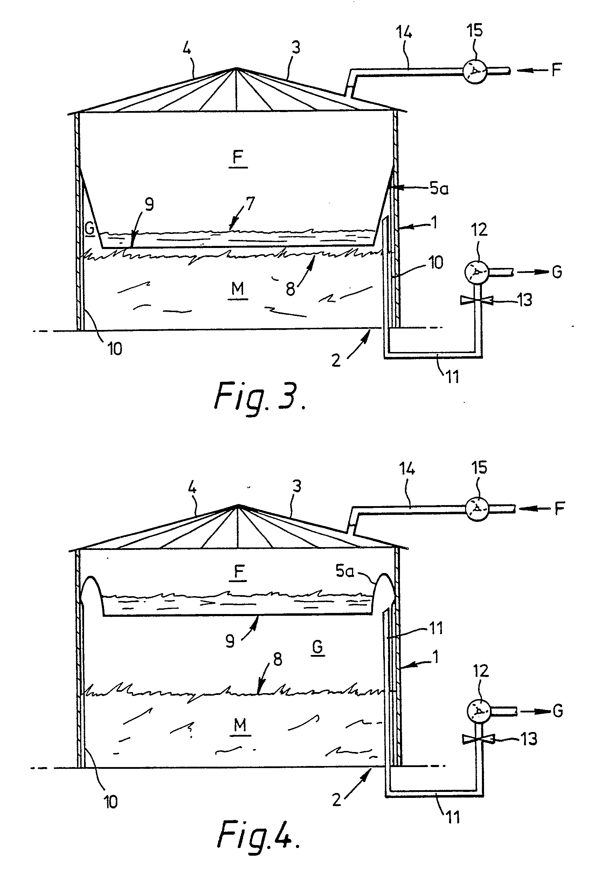

According to a second main embodiment, shown in Fig. 3 and Fig. 4, the inner wall comprises a border membrane 5a, which is also fastened with its outer edge to the inner side of the vertical silo wall 1, and a central, rigid part 9. The rigid part 9 forms together with the border membrane 5a a hollow, in which again ballast, such as water 7 can be included, in order to load the inner wall 5a, 9 in a downward direction. By the generation of the gas G from the manure with the surface 8 in the gas space below the inner wall 5a, 9 an increased pressure develops, which moves this inner wall in an upward direction, until a balanced position is reached, shown in Fig. 4. In this position the border membrane has been bent upwardly from the substantially oblique downward direction to an inverted V-shape. Also in this embodiment the means 11, 12 and 13 are present for the removal of biogas G from the gas space, as well as the fluid-pressure means 14 and 15. This embodiment is particularly suitable for narrow, high silos and for applications in which the unused space above the liquid or other ballast and the inner wall 5a, 9 must be kept as small as possible, so that the largest possible part of the silo can be used for manure and gas above it.

In both embodiments the pressure difference over the inner wall and/or the amount of ballast can be altered in a simple way, by which the pressure of the gas G can be simply controlled, such as by adding water. With the inner wall structure according to the invention an automatic centering of the ballast is reached and no separate guide elements are necessary in order to prevent the turning over and/or jamming of the inner wall against the side wall.

**Claims**

1. Tank for the storage of gas under low pressure, in particular biogas, which tank is provided with a vessel having a gas tight bottom (2) and side wall (1) of solid material, in which at the inner side of the side wall, substantially near the upper end of the side wall (1), a movable gas tight inner wall (5; 9) is gas tightly fixed, which inner wall comprises at least in the border part adjacent to the side wall a thin, flexible membrane, whereas into the gas tight gas space of variable contents (G) thus formed at least one conduit (11) for the removal of gas opens, characterized in that, a gas tight pressure space is formed above the inner wall (5; 9), said pressure space is provided with means (14) for the supply and or removal of fluid (F), which means (14) are connected to a pressure source (15), so that the inner wall (5; 9) is maintained in a floating position by the pressure difference, caused by the pressure source, between the above lying pressure space (F) and the underlying gas space (G).

2. Tank according to claim 1, characterized in that, the upper lying gas tight pressure space (F) is formed by a roof structure (3, 4) out of solid and/or gas tight material fastened on the side wall (1).

3. Tank according to claim 1 or 2, characterized in that, the inner wall (5) is substantially completely formed by the thin, flexible membrane.

4. Tank according to claim 1 or 2, characterized in that, the inner wall comprises inside the membrane border part (5a) adjacent to the side wall a rigid part (9).

5. Tank according to claim 4, characterized in that, the rigid part (9) of the inner wall is substantially flat.

6. Tank according to claims 1 through 5, characterized in that, the inner wall (5, 9) carries an amount of ballast (6, 7), such as liquid, of which the weight can be adjusted according to the pressure of the gas (G) below the inner wall (5).

7. Tank according to one or more of the claims 1—6, characterized in that, the ballast (6, 7) comprises loose solid parts, such as pieces of iron or stones.

8. Tank according to one or more of the claims 1—3 and 6 or 7, characterized in that, the membrane (5) comprises a body of revolution.

9. Tank according to one or more of the claims 1—3 and 6—8, characterized in that, the body of revolution (5) is a paraboloid.

10. Tank according to one or more of the claims 1—3 and 6—8, characterized in that, the body of revolution (5) comprises a hemisphere.

11. Tank according to one or more of the claims 1—10, characterized in that, the membrane (5) is made from plastic, such as soft PVC.

12. Tank according to one or more of the claims 1—11, characterized in that, the inner wall (5) is substantially vertically movable.

13. Tank according to one or more of the claims 1—12, characterized in that, below the membrane (5) a thin flexible skirt (10) is fastened, which skirt hangs downwardly from the fastening of the membrane to the side wall (1) along this side wall downwardly into the organic fermentable substances on the bottom of the gas space.

14. Tank according to one or more of the claims 1—12, characterized in that, as vessel a round cylindrical silo (1, 2) with conical roof (3) is used.

**Revendications**

1. Réservoir pour le stockage de gaz sous faible pression, en particulier de biogaz, ce réservoir est muni d'un récipient ayant un fond et une paroi latérale (1) étanches aux gaz en matière solide dans lequel, du côté interne de la paroi latérale, pratiquement au voisinage de l'extrémité supérieure de la paroi latérale (1), une paroi interne mobile étanche aux gaz (5, 9) est fixée de manière étanche aux gaz, cette paroi interne comprend au moins dans la partie de bord adjacente à la paroi latérale, une membrane fine, souple, tandis que dans l'espace étanche au gaz de capacité variable (G) ainsi formé débouche au moins une conduite (11) pour l'élimination du gaz, caractérisé en ce qu'un espace de pression étanche aux gaz est formé au-dessus de la paroi interne (5, 9), en ce que cet espace de pression est muni de moyens (14) pour l'arrivée et/ou l'élimination de fluide (F), ces moyens (14) sont reliés à une source de pression (15), de sorte que la paroi interne (5, 9) est maintenue dans une position flottante par la différence de pression causée par la source de pression entre l'espace sous pression (F) situé au-dessus et l'espace de gaz (G) situé au-dessous.

2. Réservoir selon la revendication 1, caractérisé en ce que l'espace de pression étanche aux gaz (F) situé au-dessus est formé d'une structure de toit (3, 4) en matière étanche aux solides et/ou aux gaz, fixée sur la paroi latérale (1).

3. Réservoir selon l'une quelconque des revendications 1 ou 2, caractérisé en ce que la paroi interne (5) est formée pratiquement complètement par la membrane fine, souple.

4. Réservoir selon l'une quelconque des revendications 1 ou 2, caractérisé en ce que la paroi interne comprend à l'intérieur de la partie bord (5a) de la membrane adjacente à la paroi latérale une partie rigide (9).

5. Réservoir selon la revendication 4, caractérisé en ce que la partie rigide (9) de la paroi interne est pratiquement plane.

6. Réservoir selon l'une quelconque des revendications 1 à 5, caractérisé en ce que la paroi interne (5, 9) porte une quantité d'un lest (6, 7), tel que du liquide, dont le poids peut être ajusté en fonction de la pression du gaz (G) au-dessous de la paroi interne (5).

7. Réservoir selon une ou plusieurs des revendications 1 à 6, caractérisé en ce que le lest (6, 7) comprend des pièces solides en vrac, telles que des morceaux de fer ou des pierres.

8. Réservoir selon une ou plusieurs des revendications 1 à 3 et 6 ou 7, caractérisé en ce que la membrane (5) comprend un corps de révolution.

9. Réservoir selon une ou plusieurs des revendications 1 à 3 et 6 à 8, caractérisé en ce que le corps de révolution (5) est un paraboloïde.

10. Réservoir selon une ou plusieurs des revendications 1 à 3 et 6 à 8, caractérisé en ce que le corps de révolution (5) comprend une hémisphère.

11. Réservoir selon une ou plusieurs des revendications 1 à 10, caractérisé en ce que la membrane (5) est fabriquée en une matière plastique telle que du PVC souple.

12. Réservoir selon une ou plusieurs des revendications 1 à 10, caractérisé en ce que la paroi interne (5) est déplaçable pratiquement verticalement.

13. Réservoir selon une ou plusieurs des revendications 1 à 12, caractérisé en ce qu'au-dessous de la membrane (5) est fixée une jupe (10) fine et souple, cette jupe pend vers le bas depuis la fixation de la membrane à la paroi latérale (1) le long de cette paroi latérale en direction du bas dans les substances organiques fermentescibles au bas de l'espace de gaz.

14. Réservoir selon une ou plusieurs des revendications 1 à 12, caractérisé en ce qu'on utilise comme récipient un silo rond cylindrique (1, 2) avec un toit conique (3).

**Patentansprüche**

1. Behälter zur Lagerung von Gas unter niedrigem Druck, insbesondere Biogas, welcher Behälter mit einem Gefäß versehen ist, das einen gasdichten Boden (2) und eine Seitenwand (1) aus festem Material hat, bei dem auf der Innenseite der Seitenwand, im wesentlichen in der Nähe des oberen Endes der Seitenwand (1), eine bewegbare, gasdichte Innenwand (5, 9) gasdicht befestigt worden ist, welche Innenwand, wenigstens im Randteil neben der Seitenwand, eine dünne, biegsame Membran aufweist, während wenigstens eine Leitung (11) in den, auf diese Weise gebildeten, gasdichten Gasraum mit variablem Inhalt (G) für die Entfernung des Gases mündet, dadurch gekennzeichnet, daß ein gasdichter Druckraum oberhalb der Innenwand (5, 9) gebildet ist, welcher Druckraum mit Mitteln (14) für die Zufuhr und/oder Entfernung des Mediums (F) versehen ist, welche Mittel (14) mit einer Druckquelle (15) verbunden sind, so daß die Innenwand (5, 9) durch den Druckunterschied, verursacht durch die Druckquelle, zwischen dem obenliegenden Druckraum (F) und dem untenliegenden Gasraum (G) in einer schwimmenden Position gehalten wird.

2. Behälter nach Anspruch 1, dadurch gekennzeichnet, daß der obenliegende, gasdichte Druckraum (F) durch eine Dachkonstruktion (3, 4) auf festem und/oder gasdichtem Material gebildet ist, das an der Seitenwand (1) befestigt ist.

3. Behälter nach Anspruch 1 oder 2, dadurch gekennzeichnet, daß die Innenwand (5) im wesentlichen vollständig aus der dünnen, biegsamen Membran gebildet ist.

4. Behälter nach Anspruch 1 oder 2, dadurch gekennzeichnet, daß die Innenwand innerhalb des Membranrandteiles (5a) nahe der Seitenwand einen starren Teil (9) aufweist.

5. Behälter nach Anspruch 4, dadurch gekennzeichnet, daß der starre Teil (9) der Innenwand im wesentlichen flach ist.

6. Behälter nach den Ansprüchen 1 bis 5, dadurch gekennzeichnet, daß die Innenwand (5, 9) eine Ballastmenge (6, 7), wie Flüssigkeit, trägt, deren Gewicht entsprechend dem Druck des Gases (G) unter der Innenwand (5) eingestellt werden kann.

7. Behälter nach einem oder mehreren der Ansprüche 1 bis 6, dadurch gekennzeichnet, daß der Ballast (6, 7) lockere, feste Teile, wie Eisen- oder Steinstückchen umfaßt.

8. Behälter nach einem oder mehreren der Ansprüche 1 bis 3 und 6 oder 7, dadurch gekennzeichnet, daß die Membran (5) einen Drehkörper umfaßt.

9. Behälter nach einem oder mehreren der Ansprüche 1 bis 3 und 6 bis 8, dadurch gekennzeichnet, daß der Drehkörper (5) ein Paraboloid ist.

10. Behälter nach einem oder mehreren der Ansprüche 1 bis 3 und 6 bis 8, dadurch gekennzeichnet, daß der Drehkörper (5) eine Halbkugel umfaßt.

11. Behälter nach einem oder mehreren der Ansprüche 1 bis 10, dadurch gekennzeichnet, daß die Membran (5) aus Kunststoff, wie weichem P.V.C., hergestellt ist.

12. Behälter nach einem oder mehreren der Ansprüche 1 bis 11, dadurch gekennzeichnet, daß die Innenwand (5) im wesentlichen senkrecht bewegbar ist.

13. Behälter nach einem oder mehreren der Ansprüche 1 bis 12, dadurch gekennzeichnet, daß unter der Membran (5) eine dünne, bewegbare Schürze (10) befestigt ist, die von der Befestigung der Membran an der Seitenwand (1), diese Seitenwand entlang, abwärts bis in die organischen, gärungsfähigen Stoffe auf dem Boden des Gasraumes hängt.

14. Behälter nach einem oder mehreren der Ansprüche 1 bis 12, dadurch gekennzeichnet, daß ein runder zylindrischer Silo (1, 2) mit konischem Dach (3) als Gefäß benutzt wird.

Fig.1.

Fig.2.

Fig. 3.

Fig.4.

2